# EUROPÄISCHE PATENTSCHRIFT

(11) **EP 0 404 998 B1**
(45) Veröffentlichungstag und Bekanntmachung des Hinweises auf die Patenterteilung: **09.11.1994**
(21) Anmeldenummer: 89111991.9
(22) Anmeldetag: 30.06.1989
(51) Int. Cl.: A61B 6/04

(54) **Patientenlagerungstisch mit in Richtung der Längsachse des Patientenlagerungstisches verstellbarer Lagerungsplatte**
Patient-supporting table having a supporting plate adjustable in the length direction of the table
Table de support pour patients à plateau de support réglable en direction de la longueur de la table

(43) Veröffentlichungstag der Anmeldung: 02.01.1991
(73) Patentinhaber: SIEMENS AKTIENGESELLSCHAFT, 80333 München (DE)
(72) Erfinder: Schäfer, Willi, Dipl.-Ing.(FH), D-8520 Erlangen (DE)

(56) Entgegenhaltungen:
- EP-A- 0 294 679
- DE-A- 2 759 079
- DE-A- 3 222 332
- US-A- 4 475 072
- US-A- 4 568 071

## Beschreibung

Die Erfindung betrifft einen Patientenlagerungstisch mit einer Lagerungsplatte, welche eine Auflagefläche für einen Patienten aufweist und mit einem die Lagerungsplatte tragenden Basisteil, wobei die Lagerungsplatte relativ zu dem Basisteil verstellbar ist.

Derartige Patientenlagerungstische werden für die unterschiedlichsten Anwendungen benötigt, in denen es erforderlich ist, einen auf der Auflagefläche der Lagerungsplatte liegenden Patienten relativ zu einem Geräteteil, z.B. einer Gammastrahlenquelle, einer Röntgenstrahlenquelle oder einem Lithotripter, verstellen zu können.

Patientenlagerungstische der eingangs genannten Art sind aus der DE-C-26 12 728 und der US-A-4 568 071 bekannt. Im Falle der bekannten Patientenlagerungstische ist jeweils die Lagerungsplatte mittels einer Anzahl von Geradeführungen, die jeweils aus einem Längsführungslager und einer Führungsstange gebildet sind, mit dem Basisteil verbunden. Auf diese Weise ist bei einer Verstellung der Lagerungsplatte relativ zu dem Basisteil eine präzise und mechanisch stabile Führung gewährleistet. Allerdings verursacht die Verwendung von Geradeführungen erhebliche Kosten. Zum einen handelt es sich nämlich bei Längsführungslagern und den für diese benötigen Führungsstangen um kostspielige Bauteile, zum anderen muß ein ganz erheblicher mechanischer Bearbeitungsaufwand getrieben werden, um einerseits sicherzustellen, daß die Führungsstangen exakt parallel zueinander verlaufen, und um andererseits sicherzustellen, daß die Längsführungslager genau miteinander fluchten und die für das Zusammenwirken mit den Führungsstangen erforderlichen Positionen exakt einnehmen. Im Falle der DE-C-26 12 728 handelt es sich bei den Längsführungslagern übrigens um Längsführungskugellager.

Der Erfindung liegt die Aufgabe zugrunde, einen Patientenlagerungstisch der eingangs genannten Art so auszubilden, daß bei möglichst geringem Kostenaufwand eine präzise und mechanisch stabile Führung der Lagerungsplatte relativ zu dem Basisteil gewährleistet ist.

Nach der Erfindung wird diese Aufgabe dadurch gelöst, daß die der Auflagefläche abgewandten Seite der Lagerungsplatte eine Eingriffsfläche darstellt, die an einer entsprechend geformten Eingriffsfläche des Basisteiles nach Art eines Gleitlagers flächenhaft anliegt und daß die Eingriffsflächen der Lagerungsplatte und des Basisteiles beim Verstellen der Lagerungsplatte relativ zum Basisteil aufeinander gleiten. Im Falle der Erfindung sind also keine besonderen Längsführungen erforderlich. Vielmehr sind sowohl die Lagerungsplatte als auch das Basisteil derart ausgebildet, daß sie nach Art eines Gleitlagers zusammenwirken können. Dies wird durch die einander entsprechende Formgebung der Eingriffsflächen auf äußerst kostengünstige Weise erreicht. Dabei ergibt sich eine weitere Verringerung der Kosten und ein geringer Fertigungsaufwand, wenn die Eingriffsflächen spanlos hergestellt werden. Die Erfindung eignet sich besonders für solche Patientenlagerungstische, deren Lagerungsplatte relativ zu dem Basisteil geradlinig, vorzugsweise wenigstens in Richtung der Längsachse des Patientenlagerungstisches, verstellbar ist. Da die Lagerungsplatte und das Basisteil im Bereich der Eingriffsflächen flächenhaft aneinander anliegen, ergibt sich eine stabile Abstützung der Lagerungsplatte. Vorzugsweise ist vorgesehen, daß die Eingriffsfläche der Lagerungsplatte wenigstens 30 % der Fläche der Unterseite der Lagerungsplatte umfaßt und daß stets wenigstens 50 % der Eingriffsfläche der Lagerungsplatte an der Eingriffsfläche des Basisteiles anliegen. Insbesondere dann kann die Lagerungsplatte als dünnwandiges schalenförmiges Bauteil ausgebildet sein, das gemäß einer bevorzugten Ausführungsform der Erfindung aus faserverstärktem Kunststoff gebildet ist. Die Ausbildung der Lagerungsplatte als dünnwandiges schalenförmiges Bauteil ist möglich, da die Lagerungsplatte eine flächenhafte Unterstützung durch das Basisteil erfährt.

Das Basisteil weist gemäß einer Variante der Erfindung einen der Gestalt der Lagerungsplatte angepaßten, im wesentlichen plattenförmigen Abschnitt auf, dessen der Unterseite der Lagerungsplatte zugewandte Oberseite die Eingriffsfläche des Basisteiles bildet. Dabei besteht die Möglichkeit, das Basisteil wenigstens im Bereich seiner Eingriffsfläche aus faserverstärktem Kunststoff zu bilden, was den Vorteil bietet, daß eine der Formgebung der Eingriffsfläche der Lagerungsplatte entsprechende Formgebung mit geringem fertigungstechnischen Aufwand erreichbar ist.

Im Interesse geringer Verstellkräfte und geringen Verschleißes ist gemäß einer Ausführungsform vorgesehen, daß wenigstens die Eingriffsfläche der Lagerungsplatte oder die Eingriffsfläche des Basisteiles durch einen reibungsmindernden Belag gebildet ist. Als Material für diesen Belag kommt z.B. Teflon (eingetragenes Warenzeichen) in Frage. Insbesondere wenn die Eingriffsflächen nicht eben ausgebildet sind, sondern Krümmungen oder Abwinkelungen aufweisen, ist es jedoch vorteilhaft, als reibungsmindernden Belag Polyäthylen vorzusehen, da dieses Material auch an gekrümmten oder abgewinkelten Flächen auf einfache Weise, nämlich durch Kleben, befestigt werden kann.

Eine Ausführungsform der Erfindung sieht vor, daß quer zur Verstellrichtung wirkende Führungsmittel vorgesehen sind, die die Lagerungsplatte relativ zu dem Basisteil führen. Derartige Führungsmittel sind für den Fall, daß die Lagerungsplatte relativ zu dem Basisteil geradlinig verschiebbar ist, mit geringem Aufwand realisierbar, wenn wenigstens das Basisteil oder die Lagerungsplatte mit wenigstens einem Führungsbord versehen ist, der an dem jeweils anderen Teil anliegt und parallel zur Verstellrichtung verläuft. Dabei können der Führungsbord und derjenige Bereich des anderen Teiles, an dem dieser anliegt, Bestandteil der jeweiligen Eingriffsflächen sein.

Ausführungsbeispiele der Erfindung sind in den beigefügten Zeichnungen dargestellt. Es zeigen:
- Fig. 1: eine perspektivische Ansicht eines erfindungsgemäßen Patientenlagerungstisches,
- Fig. 2: einen Querschnitt durch den Patientenlagerungstisch entsprechend der Linie II-II in Fig. 1,
- Fig. 3: einen Längsschnitt durch den Patientenlagerungstisch entsprechend der Linie III-III in Fig. 1,
- Fig. 4: eine perspektivische Darstellung der Einzelheit A gemäß Fig. 1 bei abgenommener Lagerungsplatte,
- Fig. 5: eine perspektivische Ansicht des Patientenlagerungstisches gemäß Fig. 1 bei gewendeter Lagerungsplatte,
- Fig. 6: eine perspektivische Ansicht eines weiteren erfindungsgemäßen Patientenlagerungstisches, und
- Fig. 7: eine perspektivische Ansicht der Einzelheit B gemäß Fig. 6 in Form einer Explosionsdarstellung.

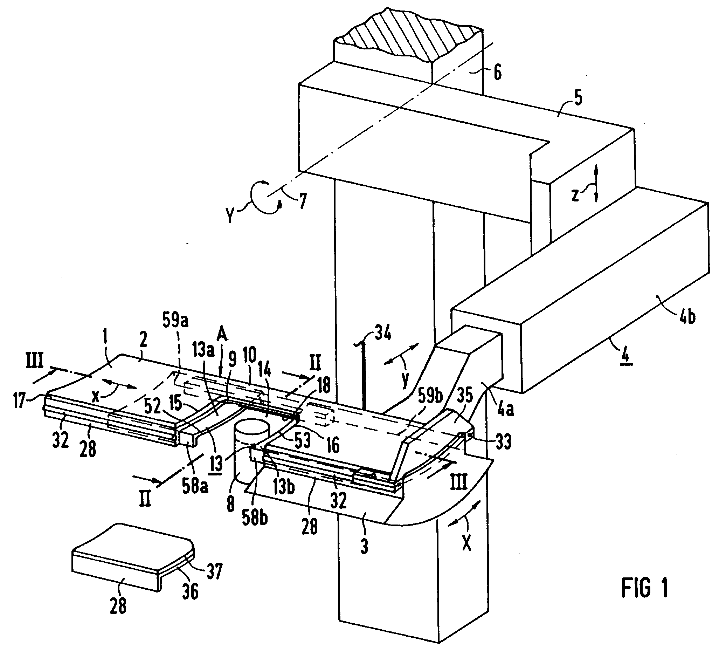

Die Fig. 1 bis 5 zeigen einen erfindungsgemäßen Patientenlagerungstisch, der Bestandteil eines Arbeitsplatzes zur Behandlung eines Patienten mit fokussierten Stoßwellen, beispielsweise zur nicht-invasiven Zertrümmerung von im Körper des Patienten befindlichen Konkrementen wie Nieren- oder Gallenblasensteinen, ist. Die fokussierten Stoßwellen werden mittels einer Stoßwellenquelle, wie sie beispielsweise in der DE-OS 33 28 051 beschrieben ist, erzeugt und in den Körper eines zu behandelnden Patienten eingeleitet, indem die Stoßwellenquelle mit ihrem durch einen flexiblen Balg gebildeten Applikationsende mit der Körperoberfläche des Patienten derart in Eingriff gebracht wird, daß eine akustische Koppelung vorliegt. Dabei werden der Patient und die Stoßwellenquelle durch entsprechendes Verstellen des Patientenlagerungstisches und der Stoßwellenquelle relativ zueinander derart positioniert, daß sich das zu zertrümmernde Konkrement im Fokus der Stoßwellen befindet. Unter der Wirkung einer Vielzahl von Stoßwellen zerfällt das Konkrement in feine Bruchstücke, die auf natürlichem Wege vom Patienten ausgeschieden werden können.

Der Patientenlagerungstisch besitzt eine Lagerungsplatte 1 mit einer Auflagefläche 2 für den Patienten. Außerdem ist ein Basisteil 3 vorgesehen, das die Lagerungsplatte 1 trägt, wobei die Lagerungsplatte 1 von dem Basisteil 3 getrennt werden kann.

Der Patientenlagerungstisch ist mit seinem Basisteil 3 derart mittels geeigneter, nicht dargestellter Gelenkmittel an einem Tragteil 4 angebracht, daß er um eine parallel zu seiner Längsachse verlaufende Achse schwenkbar ist, so wie dies durch den gekrümmten Doppelpfeil X angedeutet ist. Das Tragteil 4 weist zwei teleskopartig ineinander gefügte Armteile 4a, 4b auf, so daß der Patientenlagerungstisch quer zur Richtung seiner Längsachse in einer parallel zur Auflagefläche 2 verlaufenden Ebene in Richtung des Doppelpfeiles y verstellbar ist. Das Tragteil 4 ist mit seinem Armteil 4b an einem armförmigen Träger 5 mittels geeigneter, nicht dargestellter Führungsmittel derart befestigt, daß der Patientenlagerungstisch rechtwinklig zur Ebene seiner Lagerungsplatte 1 verstellbar ist, so wie dies durch den Doppelpfeil z angedeutet ist. Der Träger 5 ist an einem Ständer 6 um eine quer zur Längsachse des Patientenlagerungstisches und parallel zur Ebene der Lagerungsplatte 1 verlaufende Achse 7 mittels eines geeigneten, nicht dargestellten Gelenkes in Richtung des Doppelpfeiles Y schwenkbar. Außerdem ist die Lagerungsplatte 1 relativ zu dem Basisteil 3 in Richtung der Längsachse des Patientenlagerungstisches geradlinig verstellbar, so wie dies durch den Doppelpfeil x angedeutet ist.

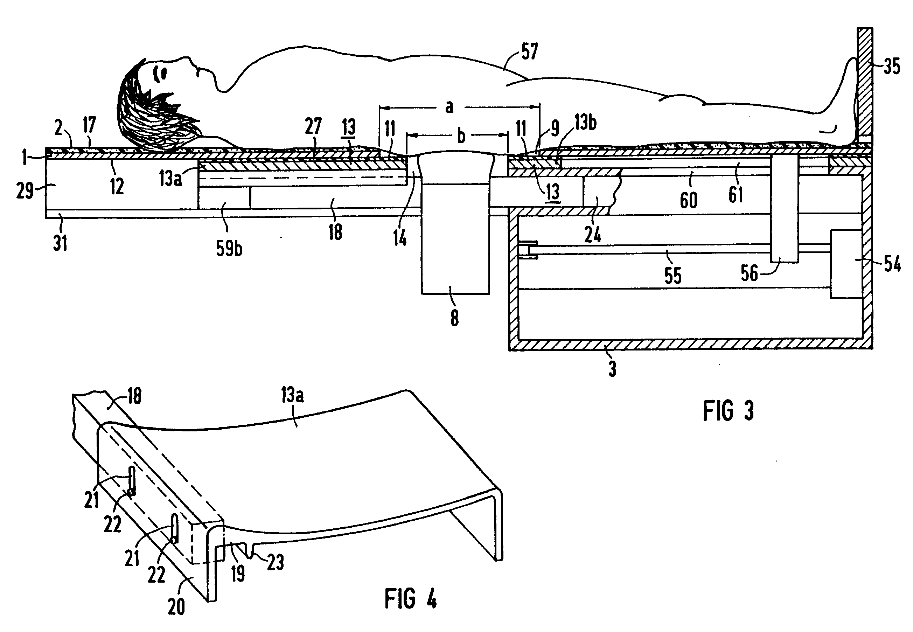

Um die beschriebenen Verstellbewegungen ausführen zu können, sind nicht dargestellte Elektromotore und geeignete Getriebe vorgesehen, die von einer Bedienperson mittels einer ebenfalls nicht dargestellten Bedieneinheit betätigbar sind. Lediglich die zum Verstellen der Lagerungsplatte 1 relativ zu dem Basisteil 3 in Richtung der Längsachse des Patientenlagerungstisches (Doppelpfeil x) vorgesehene Antriebseinrichtung ist in Fig. 3 schematisch dargestellt. Sie besteht aus einem in dem Basisteil 3 angeordneten Elektromotor 54, der eine Gewindespindel 55 antreibt. Die Gewindespindel 55 wirkt mit einer Mutter 56 zusammen, die an der Lagerungsplatte 1 in nicht dargestellter Weise lösbar befestigt ist. Dabei ist in dem Basisteil 3 ein Schlitz 60 vorgesehen, durch den die Mutter 56 mit der Lagerungsplatte 1 in Eingriff steht.

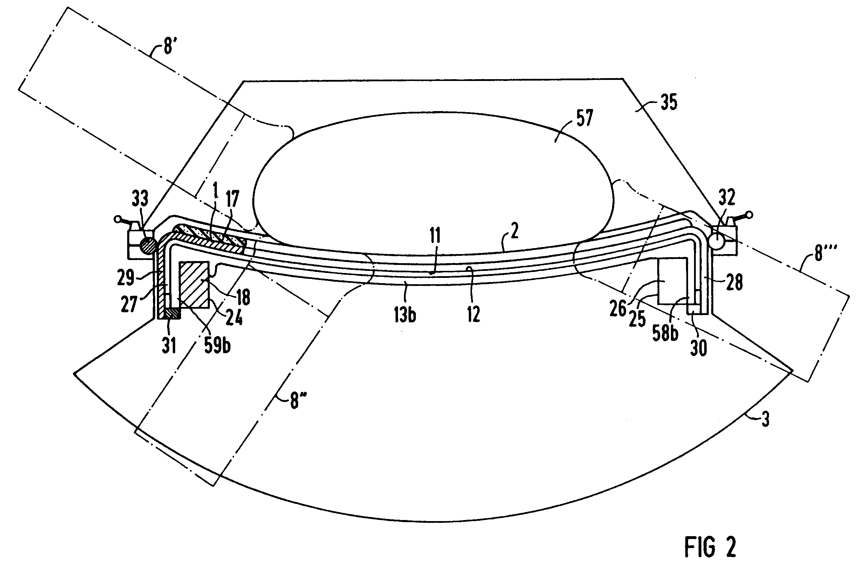

Aus den Fig. 1, 3 und 5 wird deutlich, daß der Patientenlagerungstisch mit seinem Basisteil 3 derart an dem Tragteil 4 angebracht ist, daß der Patientenlagerungstisch nur an seinem einen Ende unterstützt ist. Bei dem unterstützten Ende des Patientenlagerungstisches handelt es sich um dessen Fußende. Das freie Ende des Patientenlagerungstisches bildet dessen Kopfende. Infolge des Umstandes, daß der Patientenlagerungstisch nur an seinem einen Ende unterstützt ist, ergibt sich eine verbesserte Zugänglichkeit zu dem Patientenlagerungstisch bzw. einem auf diesem liegenden Patienten. Um eine an einer geeigneten, nicht dargestellten Halterung angebrachte Stoßwellenquelle 8, die in Fig. 1 schematisch angedeutet ist, aus möglichst beliebigen Richtungen an der Körperoberfläche eines auf der Lagerungsplatte 1 liegenden Patienten zur Anlage bringen zu können, ist die Lagerungsplatte 1 mit einem Ausschnitt versehen, der als etwa rechteckige Ausnehmung 9 in der in Fig. 1 vorderen Längskante der Lagerungsplatte 1 ausgeführt ist. Wie aus Fig. 2 anhand der strichliert dargestellten und mit 8′ und 8˝ bezeichneten Extrempositionen der Stoßwellenquelle 8 ersichtlich ist, ist nur ein relativ geringer Winkelbereich durch den im Bereich der Ausnehmung 9 verbleibenden Abschnitt 10 der Lagerungsplatte 1 versperrt. Insbesondere ist es möglich, die Stoßwellenquelle 8 von schräg unten lateral an den im Querschnitt angedeuteten Körper 57 eines auf der Lagerungsplatte 1 liegenden Patienten anzukoppeln. Diese Position der Stoßwellenquelle 8 ist ebenfalls strichliert dargestellt und mit 8‴ bezeichnet.

Um in Behandlungsfällen, die es erfordern, daß die Stoßwellenquelle 8 von der Unterseite des Patientenlagerungstisches her durch die Ausnehmung 9 mit dem Körper 57 des Patienten in Eingriff gebracht wird, eine Positionierung des Patienten relativ zu der Stoßwellenquelle 8 durch Verschieben der Lagerungsplatte 1 in Richtung der Längsachse des Patientenlagerungstisches bei bereits in der Ausnehmung 9 befindlicher Stoßwellenquelle 8 zu ermöglichen, weist die Ausnehmung 9 in Richtung der Längsachse des Patientenlagerungstisches eine lichte Weite a (Fig. 3) auf, die größer ist als dies die entsprechende Abmessung der Stoßwellenquelle 8, z.B. deren Durchmesser, erfordert. Es reicht zwar aus, wenn die Lagerungsplatte 1 bei in der Ausnehmung 9 befindlicher Stoßwellenquelle 8 in Richtung der Längsachse des Patientenlagerungstisches um etwa ± 100 mm verschiebbar ist; bei einem Durchmesser der Stoßwellenquelle von ca. 250 mm bedeutet dies jedoch, daß die Lagerungsplatte 1 allein keine ausreichende Unterstützung des Patienten im Bereich der dann eine lichte Weite a von ca. 450 mm aufweisenden Ausnehmung 9 bieten kann.

Um eine ausreichende Unterstützung des Patienten im Bereich der Ausnehmung 9 zu gewährleisten und insbesondere ein Durchrutschen des Patienten durch die Ausnehmung 9 zu vermeiden, weist das Basisteil 3 einen der Gestalt der Lagerungsplatte 1 im wesentlichen angepaßten plattenförmigen Abschnitt 13 auf, der derart unterhalb der Lagerungsplatte 1 angeordnet ist, daß seine Oberseite 11 der Unterseite 12 der Lagerungsplatte 1 zugewandt ist. Ähnlich wie die Lagerungsplatte 1 besitzt auch das Basisteil 3 einen Ausschnitt. Dieser ist ähnlich wie der Ausschnitt im Falle der Lagerungsplatte 1 als Ausnehmung 14 in der in Fig. 1 vorderen Längskante des plattenförmigen Abschnittes 13 ausgeführt. Die lichte Weite b der Ausnehmung 14 in Richtung der Längsachse des Patientenlagerungstisches ist wenig größer als der Durchmesser der Stoßwellenquelle 8 und ist somit deutlich kleiner als die lichte Weite a der Ausnehmung 9 der Lagerungsplatte 1.

Es versteht sich, daß bei von der Unterseite des Patientenlagerungstisches her mit dem Körper 57 eines Patienten in Eingriff stehender Stoßwellenquelle 8 die Lagerungsplatte 1 und das Basisteil 3 relativ zueinander so angeordnet sein müssen, daß die Ausnehmung 9 der Lagerungsplatte 1 die Ausnehmung 14 des plattenförmigen Abschnittes 13 vollständig freigibt, so wie dies in den Fig. 1 bis 3 und 5 dargestellt ist. Da die Lagerungsplatte 1, so wie dies aus den Fig. 1 bis 3 ersichtlich ist, mit den quer zur Längsachse des Patientenlagerungstisches und damit quer zur Verschieberichtung verlaufenden Begrenzungskanten 15 und 16 ihrer Ausnehmung 9 im wesentlichen spaltlos an der Oberseite 11 des plattenförmigen Abschnittes 13 anliegt, bietet die Oberseite 11 des plattenförmigen Abschnittes 13 eine wirksame zusätzliche Unterstützung des Körpers 57 des Patienten, die ein Durchrutschen des Patienten durch die Ausnehmung 9 der Lagerungsplatte 1 ausschließt. Der innerhalb der Ausnehmung 9 der Lagerungsplatte 1 befindliche Teil der Oberseite 11 des plattenförmigen Abschnittes 13 des Basisteiles 3 bildet also eine zusätzliche Auflagefläche für den Körper 57 des Patienten.

Wie aus den Figuren ersichtlich ist, ist die Auflagefläche der Lagerungsplatte mit einer Polsterauflage 17 versehen, die bis an die Begrenzungskanten der Ausnehmung 9 heranreicht. Somit ist, so wie dies aus der Fig. 3 ersichtlich ist, sichergestellt, daß unmittelbar im Bereich der Begrenzungskanten 15 und 16 der Ausnehmung 9 der Lagerungsplatte 1 ein Abstand der Körperoberfläche des Patienten von der Oberseite 1 des plattenförmigen Abschnittes 13 des Basisteiles 3 vorliegt, durch den bei Verschiebungen der Lagerungsplatte 1 relativ zu dem Basisteil 3 ein Einquetschen von Hautfalten zwischen der Lagerungsplatte 1 und dem plattenförmigen Abschnitt 13 vermieden ist.

Die Fig. 1 zeigt in Verbindung mit der Fig. 3, daß der plattenförmige Abschnitt des Basisteiles 3 zwei Plattenteile 13a, 13b aufweist, die in Richtung der Längsachse des Patientenlagerungstisches und damit in Verschieberichtung gesehen auf unterschiedlichen Seiten der Ausnehmung 14 des Basisteiles 3 angeordnet sind. Die Plattenteile 13a, 13b, deren einander zugewandte Kanten 52, 53 die Ausnehmung 14 begrenzen, sind durch einen Tragholm 18 miteinander verbunden. Der Tragholm 18 verläuft unterhalb des Bereiches 10 der Lagerungsplatte 1 entlang deren von der Ausnehmung 9 unberührten, also der in Fig. 1 hinteren, Längskante. Das Plattenteil 13b ist mit dem Basisteil 3 verbunden. Der Tragholm 18 und das an dem Tragholm 18 angebrachte Plattenteil 13a bilden also eine wirksame Unterstützung der durch die Ausnehmung 9 geschwächten Lagerungsplatte 1 im Bereich ihres freien Endes. Das Plattenteil 13a wirkt als an dem freien Ende des Tragholmes 18 angebrachter, sich quer zur Längsachse der Lagerungsplatte 1 erstreckender Tragarm, der Verwindungen der Lagerungsplatte 1 entgegenwirkt.

Anhand der Fig. 4 wird deutlich, daß das Plattenteil 13a an seinem zur Befestigung an dem Tragholm 18 vorgesehenen Ende einen etwa U-förmigen Abschnitt 19 aufweist, der den Tragholm 18, der einen etwa rechteckigen Querschnitt besitzt, von oben umgreift. Der äußere Schenkel 20 des U-förmigen Abschnittes 19 ist mit Langlöchern 21 versehen, in die bei an dem Tragholm 18 angebrachtem Plattenteil 13a an der äußeren Seitenfläche des Tragholmes 18 angebrachte Vorsprünge 22 eingreifen. Dabei liegen die Vorsprünge 22 an den unteren Begrenzungskanten der Langlöcher 21 an. Das Plattenteil 13a kann also von dem Tragholm getrennt werden, indem es an seinem freien Ende so weit angehoben wird, daß der innere Schenkel 23 seines U-förmigen Abschnittes 19 nicht mehr mit der inneren Seitenfläche des Tragholmes 18 in Eingriff steht, und indem es dann nach außen abgenommen wird. In entsprechender Weise kann das Plattenteil 13a auch wieder an dem Tragholm 18 angebracht werden. Es versteht sich, daß zum Abnehmen bzw. Anbringen des Plattenteiles 13a die Lagerungsplatte 1 von dem Basisteil 3 getrennt werden muß.

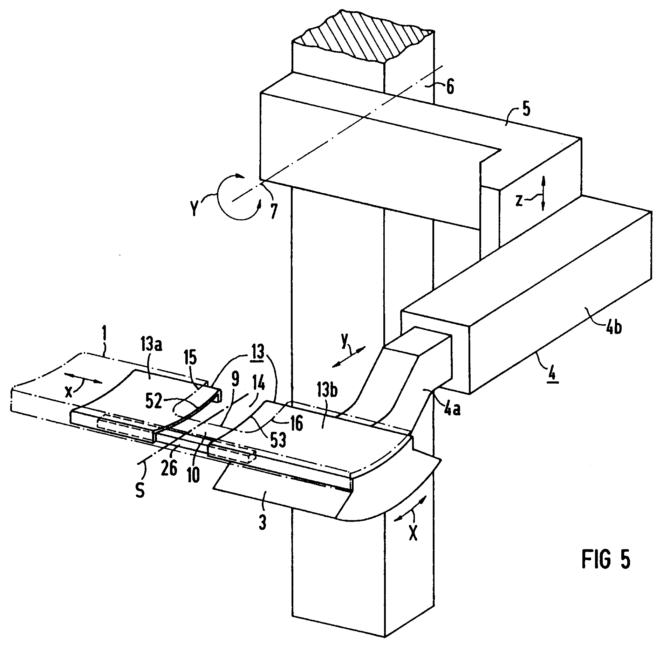

Wie insbesondere die Fig. 3 zeigt, ist der Tragholm 18 in einer entsprechenden Führungsbohrung 24 des Basisteiles 3 aufgenommen. In einer weiteren, auf der anderen Seite des Basisteiles 3 befindlichen Führungsbohrung 25 (siehe Fig. 2 und 5) ist ein weiterer Tragholm 26 aufgenommen. Die Tragholme 18, 26 sind in ihrer jeweiligen Führungsbohrung 24, 25 längsverschieblich geführt, so daß die Möglichkeit besteht, wahlweise einen der Tragholme 18, 26 aus dem Basisteil 3 auszuziehen und das Plattenteil 13a an dem jeweils ausgezogenen Tragholm 18 bzw. 26 anzubringen. Es besteht somit die Möglichkeit, den Patientenlagerungstisch ausgehend von dem Zustand gemäß Fig. 1, in dem sich die Ausnehmungen 9 und 14 in der vorderen Längskante des Patientenlagerungstisches befinden, derart zu verändern, daß sich die Ausnehmungen 9 und 14 gemäß Fig. 5 in der hinteren Längskante des Patientenlagerungstisches befinden. Hierzu ist es lediglich erforderlich, die Lagerungsplatte 1 von dem Basisteil 3 zu trennen, das Plattenteil 13a von dem Tragholm 18 zu trennen, den Tragholm 18 in die Führungsbohrung 24 einzuschieben, den Tragholm 26 aus seiner Führungsbohrung 25 auszuziehen, das Plattenteil 13a an dem Tragholm 26 anzubringen und die gewendete Patientenlagerungsplatte 1 wieder auf das Basisteil 3 aufzusetzen.

Um sicherzustellen, daß sich beim Wenden der Lagerungsplatte 1 die Lage der Ausnehmung 9 der Lagerungsplatte 1 in bezug auf die Längsachse des Patientenlagerungstisches nicht ändert, ist die Lagerungsplatte 1 zu einer quer zur Längsachse des Patientenlagerungstisches und durch die Ausnehmung 9 verlaufende Symmetrieachse S spiegelsymmetrisch ausgebildet.

Um die erwähnte Verstellbarkeit der Lagerungsplatte 1 relativ zu dem Basisteil 3 zu gewährleisten, ist die von der Auflagefläche 2 abgewandte Unterseite 12 der Lagerungsplatte 1 als Eingriffsfläche ausgebildet, die an der ebenfalls als Eingriffsfläche ausgebildeten Oberseite 11 des plattenförmigen Abschnittes 13 des Basisteiles 3 flächenhaft anliegt. Wird die Lagerungsplatte 1 relativ zu dem Basisteil 3 verstellt, gleiten die Unterseite 12 der Lagerungsplatte 1 und die Oberseite 11 des plattenförmigen Abschnittes 13 des Basisteiles 3 aufeinander. Die Lagerungsplatte 1 und der plattenförmige Abschnitt 13 des Basisteiles 3 sind einander nicht nur hinsichtlich ihrer äußeren Abmessungen, d.h. ihrer Länge und Breite, sondern auch in ihrem Querschnitt weitgehend angepaßt, so daß sich zwischen der Unterseite 11 der Lagerungsplatte 1 und der Oberseite 13 des plattenförmigen Abschnittes 13 des Basisteiles 3 eine große Anlagefläche ergibt. Die zwischen den beiden Teilen vorliegende Flächenpressung ist somit nur gering, so daß kein wesentlicher Verschleiß auftreten kann. Ebenfalls im Interesse eines geringen Verschleißes ist die Oberseite 11 des plattenförmigen Abschnittes 13 des Basisteiles 3 durch einen reibungsmindernden Belag 27 gebildet, wobei dann die der Unterseite 12 der Lagerungsplatte 1 zugewandte Seite des Belages 27 die eigentliche Eingriffsfläche des Basisteiles 3 bildet. Der reibungsmindernde Belag 27 bewirkt zugleich, daß für die Verstellung der Lagerungsplatte 1 nur eine geringe Betätigungskraft erforderlich ist. Als Material für den reibungsmindernden Belag ist ein polymerer Werkstoff, nämlich Polyäthylen vorgesehen, wodurch der Belag 27 auf einfache Weise, nämlich durch Kleben, an dem Basisteil 3 befestigt ist. Um die Mutter 56 in der erforderlichen Weise an der Lagerungsplatte 1 befestigen zu können, ist der plattenförmige Abschnitt 13 mit einem Schlitz 61 versehen, der sich durch das Plattenteil 13b und den Belag 27 erstreckt und sich mit dem Schlitz 60 des Basisteiles 3 deckt. Die Breite der Schlitze 60 und 61 ist auf das zum Durchtritt der Mutter 56 erforderliche Maß beschränkt.

Infolge der beschriebenen Ausbildung der Unterseite 12 der Lagerungsplatte 1 und der Oberseite 11 des plattenförmigen Abschnittes 13 des Basisteiles 3 als Eingriffsflächen erfährt die als dünnwandiges schalenförmiges Bauteil ausgebildete Lagerungsplatte 1 eine gleichmäßige Unterstützung, so daß Verwindungen und Durchbiegungen der Lagerungsplatte 1 nicht zu befürchten sind. Die Lagerungsplatte 1 ist aus faserverstärktem Kunststoff, vorzugsweise kohlefaserverstärktem Kunststoff, gebildet. Es ist dann auf einfache Weise möglich, die Unterseite 12 der Lagerungsplatte 1 unmittelbar auf spanlosem Wege bei der Herstellung der Lagerungsplatte 1 als Eingriffsfläche auszubilden. Auch der plattenförmige Abschnitt 13 des Basisteiles 3 ist aus faserverstärktem Kunststoff, vorzugsweise kohlefaserverstärktem Kunststoff, gebildet, so daß auch hier die Oberfläche des plattenförmigen Abschnittes 13 bereits bei dessen Herstellung durch spanlose Formgebung die Form erhält, die erforderlich ist, um nach dem Aufkleben des Belages 27 eine Oberseite 11 zu erhalten, die geeignet ist, um mit der Unterseite 12 der Lagerungsplatte 1 wie beschrieben zusammenwirken zu können.

Die Verstellbarkeit der Lagerungsplatte 1 in Längsrichtung des Patientenlagerungstisches beschränkt sich nicht auf die erwähnten ± 100 mm. Vielmehr ist, um beispielsweise endoskopische Untersuchungen durchführen zu können, eine Verstellbarkeit in Längsrichtung des Patientenlagerungstisches von insgesamt etwa 700 mm gegeben. Aus diesem Grunde ist die gesamte Unterseite 12 der Lagerungsplatte 1 als Eingriffsfläche ausgebildet. Im Falle des dargestellten Ausführungsbeispieles beträgt die Größe der Eingriffsfläche, d.h. der Oberseite 11, des plattenförmigen Abschnittes 13 des Basisteiles 3 etwa 60 % der Fläche der Eingriffsfläche der Lagerungsplatte 1. Da in beiden Extrempositionen, die die Lagerungsplatte 1 in bezug auf das Basisteil 3 einnehmen kann, die gesamte Oberseite 11 des plattenförmigen Abschnittes 13 des Basisteiles 3 an der Unterseite 12 der Lagerungsplatte 1 anliegt, ist stets eine gute Unterstützung der Lagerungsplatte 1 gewährleistet. Für den Fall, daß nicht die gesamte Unterseite 12 der Lagerungsplatte 1 als Eingriffsfläche ausgebildet ist, sollte diese wenigstens 30 % der Fläche der Unterseite 12 der Lagerungsplatte 1 umfassen. Außerdem sollte sichergestellt sein, daß stets wenigstens 50 % der Unterseite 12 der Lagerungsplatte 1 an der Eingriffsfläche des Basisteiles 3 anliegen. Die Eingriffsflächen sowohl der Lagerungsplatte 1 als auch das Basisteiles 3 müssen nicht unbedingt als durchgehende Flächen ausgebildet sein, sondern können in mehrere Abschnitte unterteilt sein, so wie dies beispielsweise im Falle der Oberseite 11 des plattenförmigen Abschnittes 13 des Basisteiles 3 infolge des Umstandes der Fall ist, daß der plattenförmige Abschnitt 13 aus den beiden Plattenteilen 13a, 13b zusammengesetzt ist.

Die Eingriffsflächen sowohl der Lagerungsplatte 1 als auch des plattenförmigen Abschnittes 13 des Basisteiles 3 erstrecken sich jeweils bis an die quer zur Tischlängsrichtung verlaufenden Begrenzungskanten 15, 16 der Ausnehmung 9 bzw. die Begrenzungskanten 52, 53 der Ausnehmung 14. Hierdurch ist die bereits erwähnte im wesentlichen spaltlose Anlage der Begrenzungskanten 15, 16 der Ausnehmung 9 an der Oberseite 11 des plattenförmigen Abschnittes 13 des Basisteiles 3 gewährleistet.

Um eine sichere Führung der Lagerungsplatte 1 relativ zu dem Basisteil 3 quer zur Längsachse des Patientenlagerungstisches, also quer zur Verschieberichtung, zu gewährleisten, ist die Lagerungsplatte an ihren beiden Längskanten mit nach unten abgewinkelten Führungsborden 28, 29 versehen, die mit ihren Innenseiten an den entsprechenden ebenfalls nach unten abgewinkelten Längskanten 58a, 59a bzw. 58b, 59b der Plattenteile 13a und 13b des plattenförmigen Abschnittes 13 des Basisteiles 3 anliegen. Dabei stellen die Innenseiten der Führungsborde 28, 29 Bestandteile der Eingriffsfläche der Lagerungsplatte 1 und die Außenseiten der nach unten abgewinkelten Längskanten 58a, 59a, 58b, 59b der Plattenteile 13a, 13b Bestandteile der Eingriffsfläche des Basisteiles 3 dar. Der Belag 27 bedeckt daher auch die Außenseiten der Längskanten 58a, 59a, 58b, 59b. Der an der mit der Ausnehmung 9 versehenen Längskante der Lagerungsplatte 1 angebrachte Führungsbord 28 weist im Bereich der Ausnehmung eine Unterbrechung auf. Die Führungsborde 28, 29 sind an den Innenseiten ihrer freien Enden mit Leisten 30, 31 versehen, die von unten an den abgewinkelten Längskanten der Plattenteile 13a, 13b des plattenförmigen Abschnittes 13 des Basisteiles 3 anliegen, wobei die Leiste 30 im Bereich der Ausnehmung 9 der Lagerungsplatte 1 unterbrochen ist. Es ist so ein sicherer Zusammenhalt der Lagerungsplatte 1 mit dem Basisteil 3 insbesondere auch beim Schwenken des Patientenlagerungstisches in Richtung des gekrümmten Doppelpfeiles X und/oder des gekrümmten Doppelpfeiles Y gewährleistet.

Um die Patientenlagerungsplatte in der zuvor beschriebenen Weise wenden zu können, muß diese also von der zur Verschiebung der Lagerungsplatte 1 in Richtung des Doppelpfeiles x dienenden Antriebseinrichtung 54, 55, 56 getrennt werden und in Richtung der Längsachse des Patientenlagerungstisches von dem Basisteil 3 abgezogen werden. Die gewendete Lagerungsplatte 1 wird anschließend in umgekehrter Richtung wieder auf das Basisteil 3 aufgeschoben und mit der erwähnten Antriebseinrichtung gekoppelt. Zur Trennung der Antriebseinrichtung 54, 55, 56 von der Lagerungsplatte 1 wird die Mutter 56 von der Lagerungsplatte 1 gelöst, indem eine nicht dargestellte, von dem Oberseite der Lagerungsplatte her zugängliche Schraube gelöst wird, die zur Befestigung der Mutter 56 an der Lagerungsplatte 1 dient.

Die Lagerungsplatte 1 ist mit entlang ihren Längskanten verlaufenden Schienen 32, 33 kreisförmigen Querschnittes versehen, die zur Befestigung von Zubehörteilen dienen. Dabei ist die an der mit der Ausnehmung 9 versehenen Längskante der Lagerungsplatte 1 angebrachte Schiene 32 im Bereich der Ausnehmung 9 unterbrochen. Die Schienen 32, 33 dienen zur Befestigung von Zubehörteilen, so wie dies in Fig. 1 am Beispiel eines Infusions-Ständers 34 und einer Fußbank 35 angedeutet ist. Der Infusions-Ständer 34 und die Fußbank 35 sind mit geeigneten Klemmvorrichtungen versehen, die eine Befestigung dieser Teile an einer gewünschten Position längs der Lagerungsplatte 1 gestatten.

Wie aus der Fig. 1 ersichtlich ist, besteht die Möglichkeit, die Ausnehmung 9 der Lagerungsplatte 1 mittels eines Verschlußteiles 36, das von den Plattenteilen 13a und 13b des plattenförmigen Abschnittes 13 des Basisteiles 3 unterstützt wird, zu verschließen, indem das Verschlußstück 36 in die Ausnehmung 9 eingelegt wird. Das Verschlußstück 36 ist mit einer Polsterauflage 37 versehen, deren Dicke der der Polsterauflage 17 entspricht.

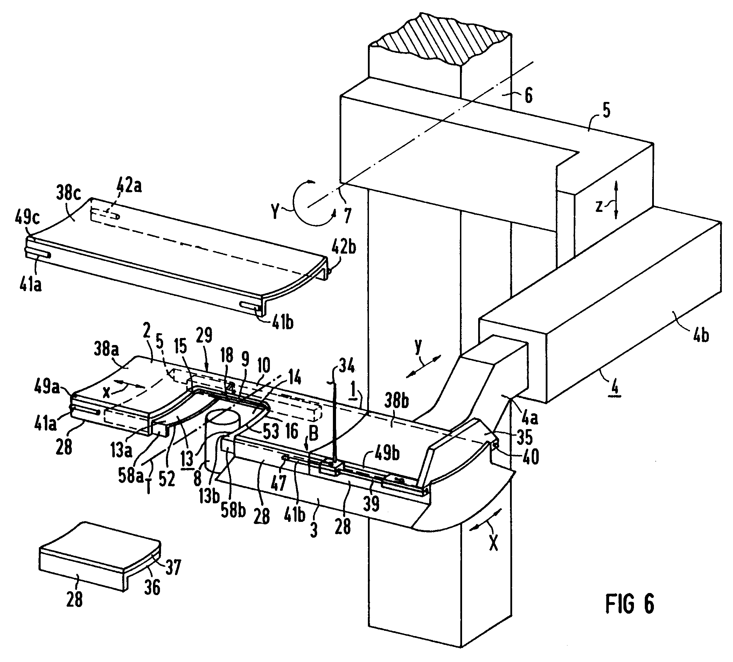

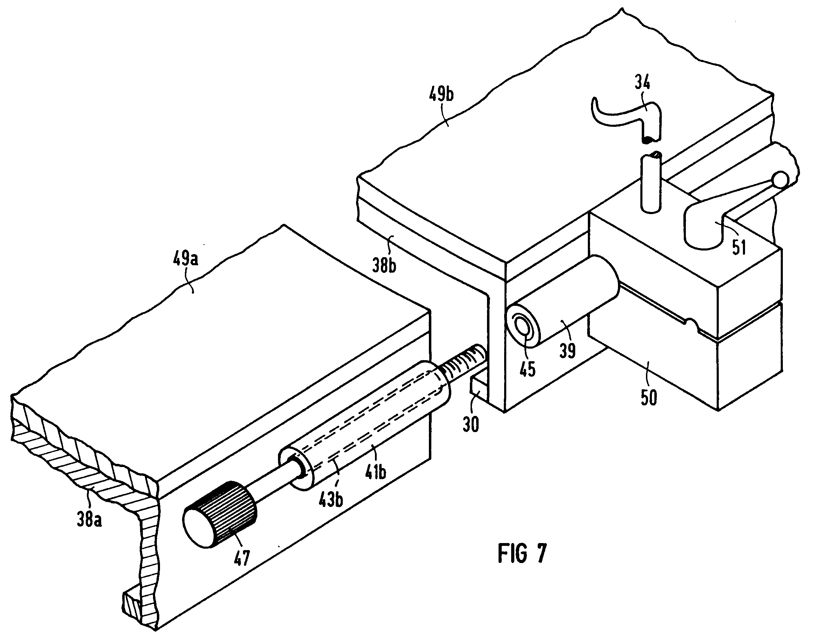

In den Fig. 6 und 7 ist ein zweites Ausführungsbeispiel eines erfindungsgemäßen Patientenlagerungstisches dargestellt, das sich von dem zuvor beschriebenen Ausführungsbeispiel lediglich durch die Ausbildung der Lagerungsplatte 1 unterscheidet, weshalb in den Fig. 6 und 7 für gleiche Teile die gleichen Bezugszeichen wie in den vorangegangenen Figuren verwendet werden. Im Falle der Fig. 6 ist die Lagerungsplatte 1 zweiteilig ausgeführt und weist die miteinander verbundenen Plattenabschnitte 38a und 38b auf. Dabei ist der das freie Ende des Patientenlagerungstisches bildende Plattenabschnitt 38a mit der Ausnehmung 9 versehen. Die nicht dargestellte zur Verschiebung der Lagerungsplatte 1 in Richtung des Doppelpfeiles x dienende Antriebseinrichtung greift mit ihrer Mutter an dem an dem Basisteil 3 angebrachten Plattenabschnitt 38b an. Der Plattenabschnitt 38b ist mit Schienen 39, 40 versehen, die entlang seiner Längskanten verlaufen und einen kreisförmigen Querschnitt aufweisen. Die Schienen 39, 40 dienen zur Befestigung von Zubehörteilen, z.B. eines Infusions-Ständers 34, sowie zur Befestigung der Fußbank 35. Im Bereich der Enden des Plattenabschnittes 38a ist dieser an seinen Längskanten 28, 29 mit Schienenabschnitten versehen, von denen in Fig. 6 nur die Schienenabschnitte 41a, 41b sichtbar sind. Die Schienenabschnitte besitzen, so wie Fig. 7 dies für den Schienenabschnitt 41b anhand der Bohrung 43b zeigt, axial durchgehende Bohrungen. An ihren von dem Basisteil 3 abgewandten Enden sind die Schienen 39, 40 in der aus Fig. 7 am Beispiel der Schiene 39 und der Gewindebohrung 45 ersichtlichen Weise mit Gewindebohrungen versehen, die bei auf dem plattenförmigen Abschnitt 13 des Basisteiles 3 aufgesetztem Plattenabschnitt 38a mit den Bohrungen der Schienenabschnitte fluchten. Wie dies aus Fig. 7 am Beispiel des Schienenabschnittes 41b mit der Bohrung 43b, der Schiene 39 mit der Gewindebohrung 45 und der Rändelschraube 47 ersichtlich ist, sind die beiden Plattenabschnitte 38a, 38b der Lagerungsplatte 1 mit Hilfe von Rändelschrauben, die sich durch die Bohrungen der Schienenabschnitte erstrecken und in die Gewindebohrungen der Schienen 39, 40 einschraubbar sind, lösbar miteinander verbunden.

Werden die Rändelschrauben entfernt, kann der Plattenabschnitt 38a axial von dem plattenförmigen Abschnitt 13 des Basisteiles 3 abgezogen, gewendet und wieder auf den plattenförmigen Abschnitt 13 das Basisteiles 3 aufgeschoben werden, so daß sich die in dem Plattenabschnitt 38a der Lagerungsplatte 1 befindliche Ausnehmung 9 wahlweise in der hinteren oder der vorderen Längskante der Lagerungsplatte 1 befinden kann. Es versteht sich, daß dann jeweils nur der geeignete Tragholm 18 bzw. 26 aus dem Basisteil 3 ausgezogen und mit dem Plattenteil 13a versehen ist. Im Gegensatz zu dem zuvor beschriebenen Ausführungsbeispiel erübrigt sich infolge der Teilung der Lagerungsplatte 1 in die zwei Plattenabschnitte 38a, 38b beim Wenden des Plattenabschnittes 38a die Trennung der erwähnten Antriebseinrichtung von der Lagerungsplatte 1. Um sicherzustellen, daß sich beim Wenden des Plattenabschnittes 38a die Lage der Ausnehmung 9 in bezug auf die Längsachse des Patientenlagerungstisches nicht ändert, ist der Plattenabschnitt 38a ähnlich wie im Falle des zuvor beschriebenen Ausführungsbeispieles die Lagerungsplatte 1 zu einer quer zur Längsachse des Patientenlagerungsrungstisches und durch die Ausnehmung 9 verlaufenden Symmetrieachse T spiegelsymmetrisch ausgebildet.

Ähnlich wie im Falle des zuvor beschriebenen Ausführungsbeispieles besteht die Möglichkeit, die Ausnehmung 9 des Plattenabschnittes 38a, beide Plattenabschnitte 38a und 38b sind übrigens mit Polsterauflagen 49a bzw. 49b versehen, durch das mit der Polsterauflage 37 versehene Verschlußstück 36 zu verschließen. Außerdem besteht, so wie dies in Fig. 6 angedeutet ist, für Behandlungsfälle, die dies gestatten, die Möglichkeit, anstelle des mit der Ausnehmung 9 versehenen Plattenabschnittes 38a einen ohne Ausnehmung ausgeführten Plattenabschnitt 38c mit dem Plattenabschnitt 38b zu verbinden. Der Plattenabschnitt 38c ist mit einer Polsterauflage 49c versehen. Im Falle des Plattenabschnittes 38c sind übrigens sämtliche vier Schienenabschnitte 41a, 41b, 42a, 42b dargestellt.

Aus der Fig. 7 ist ersichtlich, daß der Infusions-Ständer 34 mit einem klammerartigen Klemmstück 50 versehen ist, das mittels einer Klemmschraube 51 auf der Schiene 39 klemmbar ist. Die Befestigung der Fußbank 35 erfolgt in entsprechender Weise (nicht dargestellt). Auch die Befestigung des Infusionsständers 34 und der Fußbank 35 im Falle des zuerst beschriebenen Ausführungsbeispieles an den Schienen 32, 33 erfolgt in der in Fig. 7 verdeutlichten Weise.

Die Ausführungsbeispiele betreffen ausschließlich Patientenlagerungstische, die Bestandteil eines Arbeitsplatzes zur Behandlung eines Patienten mit fokussierten Stoßwellen zur nicht invasiven Zertrümmerung von im Körper des Patienten befindlichen Konkrementen sind. Es können jedoch auch Röntgenuntersuchungstische, Patientenlagerungstische für die Urologie, Lagerungstische für die Strahlentherapie usw. gemäß der Erfindung ausgebildet werden. Außerdem ist im Falle der Ausführungsbeispiele jeweils nur eine Verstellbarkeit der Lagerungsplatte 1 relativ zu dem Basisteil 3 vorgesehen, die in Richtung der Längsachse des Patientenlagerungstisches und geradlinig erfolgt. Mit den Merkmalen der Erfindung sind jedoch auch andere und/oder zusätzliche Verstellmöglichkeiten realisierbar.

## Patentansprüche

1. Patientenlagerungstisch mit einer Lagerungsplatte (1), welche eine Auflagefläche (2) für einen Patienten aufweist, und mit einem die Lagerungsplatte (1) tragenden Basisteil (3), wobei die Lagerungsplatte (1) relativ zu dem Basisteil (3) verstellbar ist, **dadurch gekennzeichnet,** daß die der Auflagefläche (2) abgewandte Unterseite (12) der Lagerungsplatte (1) selbst eine Eingriffsfläche darstellt, die an einer entsprechend geformten Eingriffsfläche des Basisteiles (3) nach Art eines Gleitlagers flächenhaft anliegt, und daß die Eingriffsflächen der Lagerungsplatte (1) und des Basisteiles (3) beim Verstellen der Lagerungsplatte (1) relativ zum Basisteil (3) aufeinander gleiten.

2. Patientenlagerungstisch nach Anspruch 1, **dadurch gekennzeichnet,** daß die Eingriffsfläche der Lagerungsplatte (1) wenigstens 30 % der Fläche der Unterseite (12) der Lagerungsplatte (1) umfaßt und daß stets wenigstens 50 % der Eingriffsfläche der Lagerungsplatte (1) an der Eingriffsfläche des Basisteiles (3) anliegen.

3. Patientenlagerungstisch nach Anspruch 1 oder 2, **dadurch gekennzeichnet,** daß die Lagerungsplatte (1) als dünnwandiges schalenförmiges Bauteil ausgebildet ist.

4. Patientenlagerungstisch nach einem der Ansprüche 1 bis 3, **dadurch gekennzeichnet,** daß die Lagerungsplatte (1) aus faserverstärktem Kunststoff gebildet ist.

5. Patientenlagerungstisch nach einem der Ansprüche 1 bis 4, **dadurch gekennzeichnet,** daß das Basisteil (3) einen der Gestalt der Lagerungsplatte (1) angepaßten, im wesentlichen plattenförmigen Abschnitt (13) aufweist, dessen der Unterseite (12) der Lagerungsplatte (1) zugewandte Oberseite (11) die Eingriffsfläche des Basisteiles (3) bildet.

6. Patientenlagerungstisch nach einem der Ansprüche 1 bis 5, **dadurch gekennzeichnet,** daß das Basisteil (3) wenigstens im Bereich seiner Eingriffsfläche aus faserverstärktem Kunststoff gebildet ist.

7. Patientenlagerungstisch nach einem der Ansprüche 1 bis 6, **dadurch gekennzeichnet,** daß wenigstens die Eingriffsfläche der Lagerungsplatte (1) oder die Eingriffsfläche des Basisteiles (3) durch einen reibungsmindernden Belag (27) gebildet ist.

8. Patientenlagerungstisch nach Anspruch 7, **dadurch gekennzeichnet,** daß als reibungsmindernder Belag (27) Polyäthylen vorgesehen ist.

9. Patientenlagerungstisch nach einem der Ansprüche 1 bis 8, **dadurch gekennzeichnet,** daß quer zur Verstellrichtung wirkende Führungsmittel vorgesehen sind, die die Lagerungsplatte (1) relativ zu dem Basisteil (3) führen.

10. Patientenlagerungstisch nach Anspruch 9, **dadurch gekennzeichnet,** daß die Lagerungsplatte (1) relativ zu dem Basisteil (3) geradlinig verstellbar ist und daß die Führungsmittel dadurch gebildet sind, daß wenigstens das Basisteil (3) oder die Lagerungsplatte (1) mit wenigstens einem Führungsbord (28, 29) versehen ist, der an dem jeweils anderen Teil (1, 3) anliegt und parallel zur Verstellrichtung verläuft.

## Claims

1. Patient support table having a support plate (1) which has a seating surface (2) for a patient, and having a base part (3) carrying the support plate (1), wherein the support plate (1) can be adjusted relative to the base part (3), characterised in that the underside (12) of the support plate (1) facing away from the seating surface (2) is itself a surface of action which bears flatly against a correspondingly shaped surface of action of the base part (3) in the manner of a sliding bearing, and in that the surfaces of action of the support plate (1) and the base part (3) slide on one another when the support plate (1) is adjusted relative to the base part (3).

2. Patient support table according to claim 1, characterised in that the surface of action of the support plate (1) comprises at least 30% of the surface of the underside (12) of the support plate (1) and in that at least 50% of the surface of action of the support plate (1) always bears against the surface of action of the base part (3).

3. Patient support table according to claim 1 or 2, characterised in that the support plate (1) is constructed as a thin-walled, shell-shaped component.

4. Patient support table according to one of claims 1 to 3, characterised in that the support plate (1) is formed of fibre-reinforced plastics material.

5. Patient support table according to one of claims 1 to 4, characterised in that the base part (3) has a substantially plate-shaped section (13) matched to the shape of the support plate (1), the upper side (11) of which section (13) faces the underside (12) of the support plate (1) and forms the surface of action of the base part (3).

6. Patient support table according to one of claims 1 to 5, characterised in that the base part (3) is formed of fibre-reinforced plastics material at least in the region of its surface of action.

7. Patient support table according to one of claims 1 to 6, characterised in that at least the surface of action of the support plate (1) or the surface of action of the base part (3) is formed by a friction-reducing coating (27).

8. Patient support table according to claim 7, characterised in that polyethylene is provided as the friction-reducing coating (27).

9. Patient support table according to one of claims 1 to 8, characterised in that guide means acting transversely relative to the direction of adjustment are provided for guiding the support plate (1) relative to the base part (3).

10. Patient support table according to claim 9, characterised in that the support plate (1) can be adjusted in a straight line relative to the base part (3), and in that the guide means are formed so that at least the base part (3) or the support plate (1) is provided with at least one guide rim (28, 29) which bears against the respective other part (1, 3), and extends parallel to the direction of adjustment.

## Revendications

1. Table de support pour patient comportant un plateau de support (1), qui possède une surface d'appui (2) pour le patient, et une partie de base (3) qui porte le plateau de support (1), le plateau de support (1) étant déplaçable par rapport à la partie de base (3), caractérisée par le fait que la face inférieure (12) du plateau de support (1), tournée à l'opposé de la surface d'appui (2), représente elle-même une surface d'engagement, qui s'applique sur une certaine étendue, à la manière d'un palier lisse, contre une surface d'engagement, de forme correspondante, de la partie de base (3) et que les surfaces d'engagement du plateau de support (1) et de la partie de base (3) glissent l'une sur l'autre lors du déplacement du plateau de support (1) par rapport à la partie de base (3).

2. Table de support pour patient suivant la revendication 1, caractérisée par le fait que la surface d'engagement du plateau de support (1) s'étend sur au moins 30 % de la surface de la face inférieure (12) du plateau de support (1) et qu'en permanence au moins 50 % de la surface d'engagement du plateau de support (1) s'appliquent sur la surface d'engagement de la partie de base (3).

3. Table de support pour patient suivant la revendication 1 ou 2, caractérisée par le fait que le plateau de support (1) est réalisé sous la forme d'un composant en forme de coque à paroi mince.

4. Table de support pour patient suivant l'une des revendications 1 à 3, caractérisée par le fait que le plateau de support (1) est formé par une matière plastique renforcée par des fibres.

5. Table de support pour patient suivant l'une des revendications 1 à 5, caractérisée par le fait que la partie de base (3) possède un élément sensiblement en forme de plaque (13), et dont la face supérieure (11), qui est tournée vers la face inférieure (12) du plateau de support (1), forme la surface d'engagement de la partie de base (3).

6. Table de support pour patient suivant l'une des revendications 1 à 5, caractérisée par le fait que la partie de base (3) est réalisée, au moins dans la zone de sa surface d'engagement, par une matière plastique renforcée par des fibres.

7. Table de support pour patient suivant l'une des revendications 1 à 6, caractériséé par le fait qu'au moins la surface d'engagement du plateau de support (1) ou la surface d'engagement de la partie de base (3) est formée par un revêtement (27) qui réduit le frottement.

8. Table de support pour patient suivant la revendication 7, caractérisée par le fait qu'on prévoit du polyéthylène pour constituer le revêtement (27) réduisant le frottement.

9. Table de support pour patient suivant l'une des revendications 1 à 8, caractérisée par le fait qu'il est prévu des moyens de guidage qui agissent transversalement à la direction de réglage et qui guident le plateau de support (1) par rapport à la partie de base (3).

10. Table de support pour patient suivant la revendication 9, caractérisée par le fait que le plateau de support (1) est déplaçable selon une trajectoire rectiligne par rapport à la partie de base (3) et que les moyens de guidage sont agencés de telle sorte qu'au moins la partie de base (3) ou le plateau de support (1) comporte au moins un bord de guidage (28,29) qui s'applique contre l'autre partie respective (1,3) et qui est parallèle à la direction de déplacement.
